(19) Europäisches Patentamt
European Patent Office
Office européen des brevets

(11) Veröffentlichungsnummer: **0 210 513**
**B1**

(12) **EUROPÄISCHE PATENTSCHRIFT**

(45) Veröffentlichungstag der Patentschrift: **23.05.90**

(51) Int. Cl.$^5$: **A 61 K 31/47**, A 61 K 31/44

(21) Anmeldenummer: **86109587.5**

(22) Anmeldetag: **14.07.86**

(54) **Zubereitungen zur Behandlung von Mycoplasmosen und bakteriellen Erkrankungen bei Geflügel.**

(30) Priorität: **24.07.85 DE 3526445**
**15.03.86 DE 3608745**

(43) Veröffentlichungstag der Anmeldung:
**04.02.87 Patentblatt 87/06**

(45) Bekanntmachung des Hinweises auf die Patenterteilung:
**23.05.90 Patentblatt 90/21**

(84) Benannte Vertragsstaaten:
**AT BE CH DE FR GB IT LI NL SE**

(56) Entgegenhaltungen:
**EP-A-0 009 425**
**EP-A-0 047 005**
**EP-A-0 049 355**
**BE-A- 863 429**
**BE-A- 870 576**
**JP-A-85 272 740**

**Die Akte enthält technische Angaben, die nach dem Eingang der Anmeldung eingereicht wurden und die nicht in dieser Patentschrift enthalten sind.**

(73) Patentinhaber: **BAYER AG**
**D-5090 Leverkusen 1 Bayerwerk (DE)**

(72) Erfinder: **Metzger, Karl Georg, Dr.**
**Pahlkestrasse 75**
**D-5600 Wuppertal 1 (DE)**
Erfinder: **Zeiler, Hans-Joachim, Dr.**
**Elsbeeker Strasse 46**
**D-5620 Velbert 15 (DE)**
Erfinder: **Scheer, Martin, Dr.**
**Herberts-Katernberg 7**
**D-5600 Wuppertal 1 (DE)**
Erfinder: **Voege, Herbert, Dr.**
**Martin-Buber-Strasse 41**
**D-5090 Leverkusen 3 (DE)**
Erfinder: **Grohe, Klaus, Dr.**
**Am Wasserturm 10**
**D-5068 Odenthal (DE)**

(56) References cited:
**JOURNAL OF CHROMATOGRAPHY, BIOMEDICAL APPLICATIONS, Band 339, Nr. 1, 1985, Seiten 214-218, Elsevier Science Publishers B.V., Amsterdam, NL; G. MONTAY et al.: "Improved high-performance liquid chromatographic determination of pefloxacin and its metabolite norfloxacin in human plasma and tissue"**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 210 513 B1

(56) References cited:
CHEMOTHERAPY, Band 32, Suppl. 3, April 1984, Seiten 70-85, Tokyo, JP;
ANTIMICROBIAL AGENTS AND CHEMOTHERAPY, Band 23, Nr. 5, Mai 1983, Seiten 641-648, American Society for Microbiology, US; S. NAKAMURA et al.: "In vitro antibacterial properties of AT-2266, a new pyridonecarboxylic acid"

ANTIMICROBIAL AGENTS AND CHEMOTHERAPY, Band 23, Nr. 3, März 1983, Seiten 509-511, American Society for Microbiology, US; Y. OSADA et al.: "Antimycoplasmal activity of ofloxacin (DL-8280)

J. ANTIMICROB. CHEMOTHER. Band 15, Nr. 6, Juni 1985, Seiten 787-789; R.J. FALLON et al.: "In-vitro sensitivity of legionellas, meningococci and mycoplasmas to ciprofoxacin and enoxacin" und M. BAKHTIAR et al.: "Human serum albumin for In-vitro tests"

HOJI et al, Proceedings and Abstracts of the 17 Worlds Poultry Congress, pp 578 - 580, Helsinki, 19844

**Beschreibung**

Die vorliegende Erfindung betrifft Zubereitungen, die Chinoloncarbonsäuren und ihre Derivate enthalten, zur Anwendung über das Trinkwasser gegen Mycoplasmen und bakterielle Erkrankungen bei Geflügel.

Es ist bereits bekannt geworden, daß Chinoloncarbonsäuren und ihre Derivate bakterizide Eigenschaften besitzen (DE—OS 3 033 157). Es ist jedoch nichts über ihre breite und universelle Anwendbarkeit bei Tieren sowie über Zubereitungen, die besonders zur Anwendbarkeit bei Tieren geeignet sind, bekannt geworden. Besondere Probleme bereitet es dabei in der Tiermedizin Infektionen zu behandeln, die durch Escherichia coli, Salmonellen oder Mycoplasmen hervorgerufen werden, vor allem wenn diese Erreger inzwischen gegen bekannte Mittel resistent geworden sind.

Die vorliegende Erfindung betrifft Zubereitungen, die Chinoloncarbonsäuren und ihre Derivate der Formel

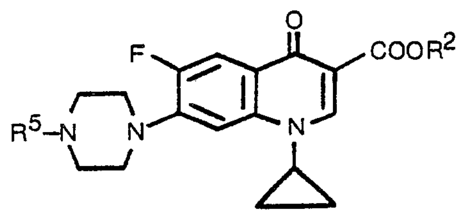

(I)

in welcher

$R^2$ für Wasserstoff, Alkyl mit 1 bis 4 Kohlenstoffatomen

$R^5$ für Wasserstoff, Methyl oder Ethyl steht, sowie ihrer pharmazeutisch verwertbaren salze,

Henthalten, die über dar Trinkwasser gegen Mycoplasmosen und bakterielle Erkrankungen bei Geflügel veräbreicht werden.

Insbesondere seien als Wirkstoffe die folgenden Chinoloncarbonsäuren und ihre Derivate gennannt:

1-Cyclopropyl-6-fluor-1,4-dihydro-4-oxo-7-(1-piperazinyl oder 4-Methyl- oder 4-ethyl-1-piperazinyl)-chinolin-3-carbonsäure, sowie die Methyl- und Ethylester dieser Verbindung.

Als pharmazeutisch verwertbare Salze seien Salze mit Säuren und Basen, die physiologisch verträgliche Salze bilden gennant. Die Salze sind bekannt oder lassen sich analog zu bekannten Verfahren herstellen.

Als Säuren seine gennant: Salzsäure, Schwefelsäure, Phosphorsäure, organische Säuren wie Ameisensäure, Essigsäure, Milchsäure, Apfelsäure, Fumarsäure, Citronensäure, Ascorbinsäure, Bernsteinsäure, Weinsäure, Malonsäure, Maleinsäure, Embonsäure.

Bevorzuge seien gennant Selzsäure, Essigsäure, Milchsäure, Embonsäure.

Als besonders bevorzugt sei Embonsäure gennant.

Als Basen seien gennant, anorganische Basen wie NaOH, KOH, $Ca(OH)_2$, Ammoniak, organische Basen wie Amine wie Mono-Di-, Trialkylamine, substituierte Amine wie Ethanolamin, cyclische Amine wie Morpholin, Piperazin, basische Aminosäure wie Arginin, Lysin, Cholin, N-Methylglucamin.

Bevorzugt sind die folgenden Basen: NaOH, KOH, Ethanolamin, Lysin, N-Merthylglucamin.

Besondere bevorzugt sind die folgenden Basen: NaOH, KOH.

Die Wirkstoffe sind bekannt oder lassen sich analog zu bekannten Verfahren herstellen.

Die Wirkstoffe werden in Form von Zubereitungen angewandt, die über das Trinkwasser verabreicht werden.

Konzentrate zur oralen Verabreichung nach Verdünnung werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden.

Als Lösungsmittel seien gennant: Physiologisch verträgliche Lösungsmittel wie Wasser, Alkohole wie Ethanol, Butanol, Benzylakohol, Glycerin, Propylenglykol, Polyethylenglykole, N-Methyl-pyrrolidon, sowie Gemische derselben.

Als Lösungsvermittler seien genannt: Lösungsmittel, die die Lösung des Wirkstoffs im Hauptlösungsmittel fördern oder sich Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Konzentrate zur Applikation über das Trinkwasser müssen so formuliert sein, daß beim Vermischen mit dem Trinkwasser eine klare Lösung oder eine stabile homogene Suspension entsteht.

Als Trägerstoffe sind daher wasserlösliche Substanzen (Futterzusatzmittel) wie Zucker oder Salze (z.B. Citrate, Phosphate, Kochsalz, Na-karbonate) geeignet.

Sie können ebenfalls Antioxydantien und Konservierungsmittel erthalten.

Die Wirkstoffe können in den Formulierungen allein oder in Mischung mit anderen Wirkstoffen, Mineralsalzen.

Spurenelementen, Vitaminen, Eiweißstoffen, Farbstoffen, Fetten oder Geschmacksstoffen vorliegen.

Die Wirkstoffe wirken gegen tierpathogene Mikroorganismen.

Zu den tierpathogenen Mikroorganismen zählen:

1. Spirochaetaceae
2. Micrococcaceae (z.B. Staphylococcen)
3. Streptococcaceae (z.B. Streptococcen der Lancefield Typen A—N)
4. Pseudomonaceaea (z.B. Ps. aeruginosa), Bordetella wie Bordetella bronchiseptica.
5. Enterobacteriaceae (z.B. Salmonella, E. coli, Klebsiella, Proteus, Citrobacter, Edwardsiella, Haemophilus, Providencia, Yersina)
6. Vibrionaceae (zz.B. Vibrio wie Vibrio cholerae, Pasteurella wie Pasteurella multocida, Aeromonas, Actinobacillus, Streptobacillus)
7. Bacetroidaceas (z.B. Bacteroides, Fusobacterium),
8. Lactobacillaceae (Erysiphylothrix, Listeria wie Listeria monocytogenes)
9. Bascillaceae (z.B. Clostridium Typen A—D, wie Clostridium perfringens), sowie anaerobe Coccen wie z.B. Peptostreptococcen und Peptococcen
10. Coryneforme Bakterien (z.B. Corynebacterium pyogenes)
11. Mycobacteriaceae (z.B. Mycobacterium avium, M. tuberculosis)
12. Nocardiaceaea (z.B. Nocardia asteroides)
13. Chlamydiaceae (z.B. Chlamydia psittace)
14. Mycoplasmataceae (z.B. M. gallisepticum).

Tierpathogene Mikroorganismen können als Mono- oder Mischinfektionen Krankheitsbilder bei folgenden Organsystemen der Tiere hervorrufen:

Lunge und Trachialraum, Verdauungssystem, Genitalsystem, Weichteile, aktiver und passiver Bewegungsapparat, Urogenitalsystem, Nervensystem, Gehörapparat, Augen.

Wie bereits erwähnt werden die Wirkstoffe zur Bekämpfung bakterieller Erkrankungen bei Geflügel verwendet.

Zu den bakteriellen Erkrankungen zählen z.B. bei Geflügel (Huhn, Pute, Perlhuhn, Wachtel, Taube, Ziervögel u.a.) Mycoplasmose, E-coli-Infektion, Chronic Respiratory Disease, Salmonellose, ansteckender Geflügelschnupfen, Pasteurellose, Campylobacter-Infektion, Psittakose-Ornithose sowie Infektionen oder Mischinfektionen mit z.B. Escherichia coli, Salmonella spp., Kliebsielle spp., Proteus spp., Haemophilus spp., Pasteurella spp., Actinobacillus spp., Pseudomonas spp., Brucella spp., ordetella spp., Moraxella spp., Campylobacter spp, Staphylococcus spp., Streptococcus spp., Listeria spp., Erysipelothrix spp., Cornynebacterium spp., Fusobacterium spp., Borellia spp., Treponema spp., Leptospira spp., Clostridium spp., Mycoplasma spp., Nocardia spp., Rickettsia spp., Mycobacterium spp., Yersinia spp.

Die Behandlung von Geflügelerkrankungen, besonders durch E.coli, Mycoplasmen, Salmonellen, Pasteurellen, Campylobacter, erfolgt durch orale Therapie im Trinkwasser, 10 bis 500 mg/l, bevorzugt 2,5 bis 100 mg/l, über 1 bis 20, bevorzugt 3 bis 10 Tage, gegenbenenfalls wiederholt (Propyhylaxeprogramm).

Zubereitungen die die oben angegebenen Wirkstoffe enthalten, eignen sich besonders zur Behandlung von Infektionen, die durch E. coli, Salmonellen und/oder Mycoplasmen hervorgerufen werden. Sie sind besonders geeignet im Einsatz gegen solche Erreger infektiöser Erkrankungen, die gegen derzeit bekannte Mittel resistent geworden sind.

Besonders genannt sei die Eignung von Zubereitungen die die oben angegebenen Wirkstoffe enthalten zur Behandlung von Salmonellosen und Mycoplasmeninfektionen beim Geflügel insbesondere bei Hühnern und Masthühnern.

Besonders hervorzuheben ist die einfache und günstige Behandlung von Salmonellen- und Mycoplasmenerkrankungen des Geflügels, insbesondere der Hühner, über das Trinkwasser. Die Wirkstoffe sind im Trinkwasser entweder in Form ihrer Salze mit wasserslöslichen Säuren oder in Form ihrer Salze mit wasserlöslichen Basen gelöst.

Die Basen bzw. Säuren werden zur Salzbildung den Wirkstoffen bevorzugt in überäquimolaren Mengen zugesetzt, so daß die anwendungsfertige wäßrige Lösung oder sauer reagiert.

Die anwendungsfertigen Lösungen enthalten bevorzugt 10—50 ppm, besonders bevorzugt 25—100 ppm, ganz besonders bevorzugt 50—100 ppm Wirkstoff.

Der pH-Wert der anwendungsfertigen sauren Lösung liegt zwischen 2 und 5, bevorzugt zwischen 3 und 5.

Der pH-Wert der anwendungsfertigen besischen Lösung liegt zwischen 8 und 11, bevorzugt zwischen 9 und 10.

## BEISPIELE

In den folgenden Beispielen werden als Wirkstoffe 1-Cyclopropyl-6-fluor-1,4-dihydro-4-oxo-7(4-ethyl-1-piperazinyl-)chinolin-3-carbonsäure (I) und 1-Cyclopropyl-6-fluor-1,4-dihydro-4-oxo-7-(1-piperaziyl)-chinolin-3-carbonsäure (II) eingesetzt.

A. Allgemeine Vorschrift zur Herstellung basisch reagierender Konzentrate zum Einsatz im Trinkwasser:

Der Wirkstoffe und die Hilfsstoffe werden in der größeren Menge des Wassers suspendiert. Es wird vorsichtig unter Rühren das Alkali dazugegeben bis der Wirkstoff in Lösung gegangen ist. Es Wird so lange weitergerührt, bis ein klares, dünnflüssiges Gel ohne Klumpen entstanden ist.

Beispiel 1

| | |
|---|---|
| Wirkstoff I | 2,500 g |
| Benzylalkohol | 1,400 g |
| Methyl-hydroxypropyl-cellulose | 3,500 g |
| Kaliumhydroxid bis pH 11 ca. | 0,004 g |
| Wasser entmineralisiert | 95,996 g |
| | 100 ml ≙ 100,900 g |

Beispiel 2

| | |
|---|---|
| Wirkstoff II | 2,00 g |
| Polyacrylsäure Na-salz | 0,62 g |
| Natronlauge 1n | 12,00 g |
| Benzylalkohol | 1,00 g |
| Wasser entmineralisiert | 85,08 g |
| | 100 ml ≙ 100,70 g |

Beispiel 3

| | |
|---|---|
| Wirkstoff I | 20,00 g |
| Benzylalkohol | 1,00 g |
| Kalilauge 10% | 30,56 g |
| Wasser entmineralisiert | 55,94 g |
| | 100 ml ≙ 107,50 g |

B. Allgemeine Vorschrift zur Herstellung sauer reagierender Konzentrate zum Einsatz im Trinkwasser:

Beispiel 4

| | |
|---|---|
| Wirkstoff I | 10,0 kg |
| Essigsäure | 2,5 kg |
| Benzylalkohol | 1,0 kg |
| Wasser entmineralisiert | 89,7 kg |
| | 100 1 ml ≙ 103,2 kg |

In einem Kessel wird Wasser vorgelegt und die übrigen Sunstanzen unter Rühren zugefügt.

Beispiel 5

| | |
|---|---|
| Wirkstoff II | 5,0 kg |
| Milchsäure 20% | 10,0 kg |
| Wasser entmineralisiert | ad 100 1 |

Die Herstellung erfolgt wie in Beispiel 4 angegeben.

In den folgenden Anwendungsbeispielen wird die hervorragende Eignung der oben genannten Wirkstoffe zur Behandlung von Erkrankungen beim Geflügel über das Trinkwasser gezeigt.

Beispiel A

Behandlung von Mycoplasmosen des Geflügels

Je 40 Küken die frei von Mycoplasma gallisepticum und Mycoplasma synoviae waren, wurden am zweiten Lebenstag mit einem pathogen Wildstamm von Mycoplasma gallisepticum infiziert. 24 Stunden nach der Infketion begann die Behandlung. Eine Gruppe von 40 Küken wurde als Kontrolle nicht behandelt. Es wurde an 5 aufeinanderfolgenden Tagen nach der Infektion über dads Trinkwasser behandelt. 19 Tage nach der Infektion wurden die Tiere getötet, gewogen und untersucht. Dabei wurden folgende Ergebnisse erhalten:

TABELLE I

| Trinkwasser Behandlung mit | Durchschnittsgewicht der Tiere nach 0 | 14 | 19 | klinische Symptome |
|---|---|---|---|---|
| Verbindung I 250 ppm | 47,3 | 355,7 | 463,8 | keine |
| Vergleichssubstanz Tylosin 500 ppm | 48,4 | 311,9 | 426,3 | deutlich, kein Tier tot |
| unbehandelt | 48,2 | 269,6 | 347,4 | deutlich, 18 Tiere tot |

Aus keinem der behandelten Tiere konnten nach Abtötung Mycoplasmen isoliert werden.

Beispiel B

Der Test wurde auf Mikrotiterplatten durchgeführt. Es wurden Konzentrationsreihen der Wirkstoffe in 0,01 N Sodalösung hergestellt. Die Konzentrationen betrugen von 256 µg/ml 0,0156 µg/ml.

Jeweils 0,5 ml einer Wirkstofflösung wurden in eine Vertiefung der Mikrotiterplatte gegeben. Dazu wurden 0,5 ml eines Inoculums von Mycoplasmen mit ca. $10^6$ Keimen pro ml gegeben.

Das Inoculum bestand aus einer modifizierten Mycoplasmen Bouillon (PPLO) die ca. 20% Pferdeserum enthielt und in der Mycoplasmen bis zu einem Titer von ca. $10^7$ Keimen pro ml (bestimmt nach der Methode von Mac Crady) herangezogen wurden.

Danach wurde die Mikrotiterplatte bei 37°C bebrütet und das Wachstum nach jeweils 48, 72 und 96 Stunden bestimmt. Es wurde als MHK-Wert die Wirkstoffkonzentration angegeben durch die eine Vermehrung der inokulierten Mycoplasmen verhindert wurde.

TABELLE II

| Wirkstoffe | MHK-Werte bei Mycoplasmenstämmen Nr. 348 | 7981 | 6442 | 4587 | 81115 | 8008 |
|---|---|---|---|---|---|---|
| Verbindung I | 0,5 | 0,5 | 0,15 | 0,5 | 0,5 | 0,25 |
| Vergleich: Nalidixinsäure | 128 | 128 | 128 | 128 | 128 | 128 |
| Flumequin | 256 | 256 | 256 | 256 | 256 | 256 |

**Patentanspruch**

Verwendung von Chinoloncarbonsäuren und ihren Derivaten der Formel I

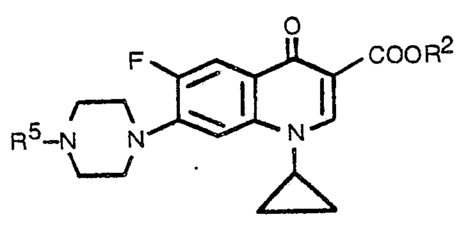

in welcher

$R^2$ für Wasserstoff oder Alkyl mit 1 bis 4 Kohlenstoffatomen

$R^5$ für Wasserstoff, Methyl oder Ethyl steht, sowie ihrer pharmazeutisch verwertbaren Salze, zur Herstellung von Mitteln, die über das Trinkwasser varabreicht werden, gegen Mycoplasmosen und bakterielle Erkrankungen bei Geflügel.

**Revendication**

Utilisation d'acides quinolone-carboxyliques et de leurs dérivés répondant à la formule I

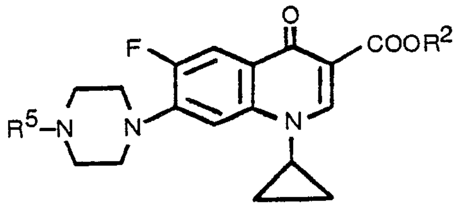

dans laquelle

$R^2$ représente l'hydrogène ou un groupe alkyle de 1 à 4 atomes de carbone

$R^5$ représente l'hydrogène, un groupe méthyle ou éthyle, et de leurs sels acceptables pour l'usage pharmaceutique, pour la préparation de médicaments, administrés par l'intermédiaire de l'eau de boisson, contre les mycoplasmoses et les maladies bactériennes des volailles.

**Claim**

Use of quinolonecarboxylic acids and derivatives thereof of the formula I

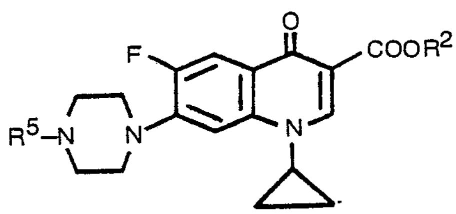

in which

$R^2$ represents hydrogen or alkyl with 1 to 4 carbon atoms,

$R^5$ represents hydrogen, methyl or ethyl, as well as the pharmaceutically utilizable salts thereof, for the preparation of agents, which can be administered in the drinking water, against mycoplasmoses and bacterial diseases in poultry.